# EUROPEAN PATENT APPLICATION

(11) **EP 2 657 242 A2**
(43) Date of publication of application: **30.10.2013**
(21) Application number: 11850909.0
(22) Date of filing: 16.12.2011
(51) Int. Cl.: C07F 9/6593, A61K 31/337, A61P 35/00

(54) **AMPHIPHILIC CYCLIC PHOSPHAZENE TRIMER, HYDROPHOBIC PHARMACEUTICAL FORMULATION MICELLIZED BY AMPHIPHILIC CYCLIC PHOSPHAZENE TRIMER, AND PREPARATION METHODS THEREOF**

(30) Priority: 20.12.2010 KR 20100130998; 09.09.2011 KR 20110091796
(71) Applicant: CNPharm Co., Ltd., Seoul 120-750 (KR)
(72) Inventor: SOHN, Youn Soo, Seoul 135-905 (KR); JUN, Yong Joo, Seoul 142-742 (KR)
(74) Representative: Vetter, Christoph
(86) International application number: PCT/KR2011/009726
(87) International publication number: WO 2012/086964

(57) **Abstract**

The present invention relates to an amphiphilic cyclic phosphazene trimer which is biocompatible and a method for preparing the same. The present invention also relates to pharmaceutical formulations of hydrophobic drugs that are micelle-encapsulated by the amphiphilic cyclic phosphazene trimer and a method for preparing the same.

## Description

### Technical Field

The present invention relates to an amphiphilic cyclic phosphazene trimer which is biocompatible and suitable for use in a drug delivery system for intravenous injection.

The present invention also relates to a pharmaceutical formulation of hydrophobic drugs by micelle-encapsulation using the amphiphilic cyclic phosphazene trimer, and a method for preparing the same. This application claims the benefit of Korean Patent Application No. 10-2010-0130998, filed on December 20, 2010, and Korean Patent Application No. 10-2011-0091796, filed on September 9, 2011, which are hereby incorporated by reference in their entireties into this application.

### Technical Field

There are various drug administration methods depending on administration routes in the body, with oral and injection administrations being prominent, inter alia. Particularly, injection administration is widely applied to incurable diseases, such as cancer and diabetes, which require effective chemotherapy, because it has an advantage over oral administration in that it can reduce the loss and uptake time of drugs by omitting the gastro-intestinal absorption step.

The drug for injection administration should be water soluble, but many therapeutics for incurable diseases are water insoluble. Thus, various surfactants are used as additives for solubilizing the water-insoluble drugs, but they provoke various side effects, with consequent limitations of therapeutic effects.

Docetaxel is one of the most widely used anticancer agents, but its water solubility is as low as 6∼7 µg/mL. Therefore, docetaxel is formulated with the surfactant polysorbate 80 in 13 % ethanol, and the formulated docetaxel is commercially available under the trade name Taxotere®. Taxotere® exhibits not only neurotoxicity due to docetaxel itself but also exerts side effects attributed to the polysorbate 80 such as anaphylaxis, edema, etc. In addition, because docetaxel has poor stability to light and heat, it is marketed in a set of two vials containing a solution of docetaxel in polysorbate 80, and a 13 % ethanol solution. For application to patients, these two solutions must be homogeneously mixed, and diluted in saline. The dilution must be infused within 8 hrs after the preparation thereof.

Propofol, a widely used hypnotic anesthetic agent, is insoluble in water, and has been commercially available under the brand name Diprivan®, which is a 1 % propofol lipid emulsion, since 1986. However, the propofol formulation in the form of a lipid emulsion comprising soybean oil causes severe pain and allergy, but no alternative formulation of propofol to avoid such side effects have been developed so far.

Recently it has been known that when small molecular hydrophobic drugs micelle-encapsulated using amphiphilic linear block copolymers as polymer drug carriers are administered by intravenous injection, the drug metabolism, biodistribution, pharmaceutical efficacy and toxicity can be remarkably improved, which belongs to "polymer therapy". Consequently, extensive studies have been directed at so-called "polymer therapy" to improve therapeutic effects of small molecular weight drugs using such a polymer drug carrier (R. Haag, F. Kratz, Angew. Chem. Int. Ed. 45, (2006) 1198-1215).

Representative polymeric micelles studied for docetaxel formulation are illustrated as in the following: a copolymer of the hydrophilic poly(ethylene oxide) and the hydrophobic poly(styrene oxide) (PEO-b-PSO) and a copolymer of poly(ethylene oxide) and poly(butylene oxide) (PEO-b-PBO) (E. Mahmud, et al. Macromolecules, 2007, 8, 2250-2257), a copolymer of hydrophilic methoxy polyethylene glycol (MPEG750) and hydrophobic oligo-caprolactone (MPEG750-b-OCL₅) (M.G. Carstens, et al. Eur. J. Pharm. Biopharm. 68 (2008) 596-606), and an amphiphatic polymer composed of hydrophilic polyethylene glycol (PEG) and hydrophobic polylactide (PLA) (PEG-b-PLA) (H.-C. Shin et al. J. Control. Release, 140 (2009) 294-300). Reviewing these studies, these amphiphilic linear block copolymer micelles exhibit partially improved properties compared to the conventional surfactant micelles. However, it was found that these amphiphilic linear block copolymer micelles are still disadvantageous in that, although micelles of the pure polymer itself are highly stable, they become unstable after being loaded with docetaxel, so that most of the drug loaded is precipitated within 24 hrs.

Most of the conventional organic amphiphilic polymers are liner block copolymers composed of linear hydrophilic and hydrophobic polymer segments and readily form micelles in aqueous solutions by self-assembly. However, these polymeric micelles are poor in stability because the intermolecular hydrophobic interaction among the hydrophobic segments of the unit block copolymer molecules is not strong enough to form strong micelle core. Particularly, when docetaxel is loaded into the micelle core, the micelles become unstable and cannot hold the drug molecules. Hence, they are unsuitable for use as drug carriers for injection. In addition to the stability of micelles, one more important physical property is required for using amphiphilic polymers as a drug carrier for injection. In general, amphiphilic polymers exist as homogenous micelles in an aqueous solution, but when the solution is heated, the polymers are separated as a solid phase at a certain temperature where the hydrogen bonding between the hydrophilic shell of the micelles and the solvent water molecules is weakened to cause the polymer micelles to be entangled with each other. This phase transition temperature is called "lower critical solution temperature (LCST)". In order to use as a safe drug carrier for intravenous injection, a polymer must has an LCST that is much higher than the body temperature (37°C) to prevent blood coagulation attributed to the phase transition of the polymer micelles.

All in all, the ideal drug carrier for intravenous injection should not only be able to form stronger and more stable micelles in an aqueous solution than the conventional surfactant micelles and linear block copolymer micelles, thereby sufficiently solubilizing and stabilizing hydrophobic drugs but also have much higher LCST (for example, 50°C) than the body temperature so as to enable safe intravenous injection, and it should improve the drug efficacy, toxicity and metabolism as well.

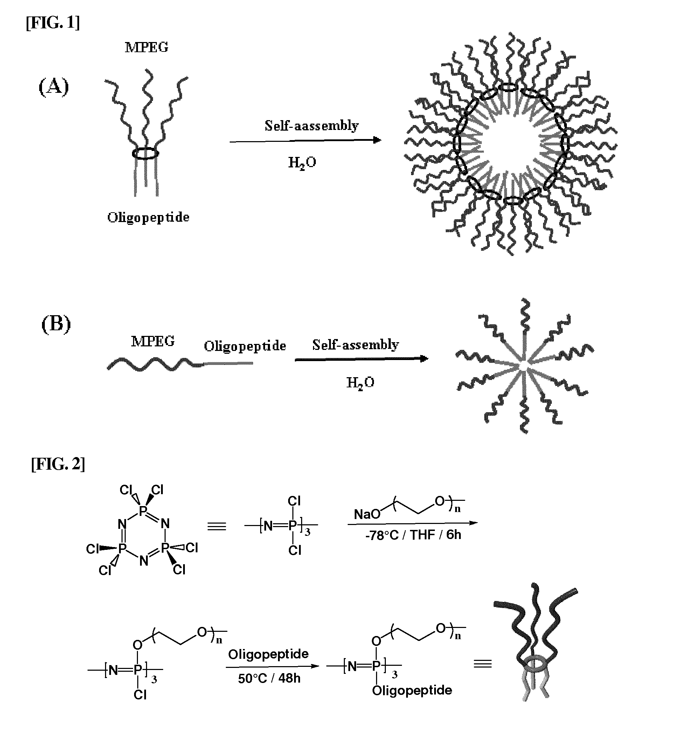

Recently the present inventors have developed a novel amphiphilic cyclic phosphazene trimer [N=P(X)(Y)]₃ in which equimolar hydrophilic polyethylene glycol (X) and hydrophobic tri- to pentapeptide (Y) are grafted into a cyclic phosphazene template in *cis*-nongeminal way, said polyethylene glycol composed of polyethylene oxide ((CH₂CH₂O)ₙ) in which the degree of polymerization (n) ranges from 7 (average molecular weight 350) to 16 (average molecular weight 750) (Korean Patent No. 10-0567397). Unlike conventional linear amphiphilic polymers, these cyclic phosphazene trimers have three hydrophobic peptide groups per molecule which are arranged in the same direction affording strong intra- and intermolecular hydrophobic interactions resulting in a strong micelle core with polyethylene glycol shell by self-assembly in aqueous solution (FIG. 1). These amphiphilic cyclic phosphazene trimers were found to be suitable for local drug delivery since they form a gel or a precipitate at body temperature and thus can afford controlled drug release upon subcutaneous injection. However, these phosphazene trimers are not suitable for use as drug carriers for intravenous injection because their LCST is around body temperature (31-42°C), which may cause blood coagulation when they are intravenously injected as described above.

### Disclosure

### Technical Problem

It is therefore an object of the present invention to provide an amphiphilic cyclic phosphazene trimer available for use as a drug carrier for intravenous injection.

It is another object of the present invention to provide pharmaceutical formulations of hydrophobic drugs that are micelle-encapsulated by the amphiphilic cyclic phosphazene trimer, and a method for preparing the same.

### Technical Solution

In order to accomplish the above objects, the present invention provides an amphiphilic cyclic phosphazene trimer represented by the following Chemical Formula 1:

[Chemical Formula 1]

[N=P(MPEG) (oligopeptide ester)]

wherein

MPEG is a methoxy polyethylene glycol with an average degree of polymerization (n) of ethylene oxide (CH₂CH₂O)ₙ ranging from 17 (average molecular weight 780) to 45 (average molecular weight 2000), and the oligopeptide ester is a C₁₋₆ alkyl ester or C₇₋₁₃ arylalkyl ester of a hexapeptide or nanopeptide.

The present invention also provides a pharmaceutical formulation of hydrophobic drugs in which a hydrophobic drug is micelle-encapsulated by the amphiphilic cyclic phosphazene trimer of Chemical Formula 1.

Further, the present invention provides a method for preparing a pharmaceutical formulation of hydrophobic drugs that are micelle-encapsulated by an amphiphilic cyclic phosphazene trimer, comprising dissolving 100 parts by weight of an amphiphilic cyclic phosphazene trimer and 2 ∼ 30 parts by weight of a hydrophobic drug in a common solvent, removing the common solvent by evaporation, and drying the residual solvent under vacuum to obtain a formulated hydrophobic drug micelle-encapsulated by the amphiphilic cyclic phosphazene trimer.

### Advantageous Effect

Unlike the known cyclic phosphazene trimers above-mentioned, which are produced in a liquid phase, the new cyclic phosphazene trimers with extended amphiphilicity according to the present invention is obtained as a solid phase. In addition, these new cyclic phosphazene trimers can afford to solubilize hydrophobic drugs such as water-insoluble small molecular anticancer agents, e.g., docetaxel effectively by micelle-encapsulation, and such micelle-encapsulated drugs are obtained in solid phase, e.g., a powder. Therefore, the amphiphilic cyclic phosphazene trimer of the present invention can bring about a remarkable improvement in the physicochemical stability, pharmaceutical efficacy and toxicity of the drug. Particularly, when a hydrophobic drug such as docetaxel is administered as a formulation micelle-encapsulated by the amphiphilic cyclic phosphazene trimer of the present invention, the drug is released in a sustained behavior with significantly improved bioavailability and mitigated toxicity. Moreover, since docetaxel can be formulated in a solid phase by micelle-encapsulation using the cyclic phosphazene trimer of the present invention, it has been found that its photo-stability could be remarkably improved unlike the conventional liquid phase formulations, so that it can be stored for a long period of time and can be conveniently used.

### Brief Description of the Drawings

FIG. 1 illustrates a structural difference between the inventive cyclic phosphazene trimer micelles and conventional linear polymer micelles.

FIG. 2 is a reaction scheme illustrating the synthesis of an amphiphilic cyclic phosphazene trimer bearing equimolar hydrophilic and hydrophobic groups. In the reaction scheme, n is an integer large enough to set the average molecular weight of methoxy polyethylene glycol between 780 and 2,000.

FIG. 3 is a conceptual view illustrating the formation of an amphiphilic cyclic phosphazene trimer micelle entrapping hydrophobic docetaxel therein.

FIG. 4 shows the results of nude mouse xenograft trial against the breast cancer line MDA-MB-231.

### Best Mode

In accordance with an aspect thereof, the present invention addresses an amphiphilic cyclic phosphazene trimer represented by the following Chemical Formula 1.

[Chemical Formula 1]

[N=P(MPEG) (oligopeptide ester)]₃

wherein

MPEG is a methoxy polyethylene glycol with an average degree of polymerization (n) of ethylene oxide (CH₂CH₂O)ₙ ranging from 17 (average molecular weight 780) to 45 (average molecular weight 2000), and the oligopeptide ester is a C₁₋₆alkylester or C₇₋₁₃arylalkylester of a hexapeptide or a nanopeptide.

The amphiphilic cyclic phosphazene trimers, previously reported by the present inventors, which are composed of the hydrophilic polyethylene glycol with an average molecular weight in the range of 350 ∼ 750 and the hydrophobic tri- to pentapeptide, are not suitable for use as drug carriers for intravenous injection because their LCST is around body temperature (31-42°C) as above-mentioned. Particularly, the micelles of these trimers, when loaded with docetaxel, are not sufficiently stable.

Keeping in mind the fact that phosphazene trimer micelles must have high stability in order to be used as a drug carrier for intravenous injection, the present inventors have performed intensive experiments using a dynamic light scattering method for structural optimization of the cyclic phosphazene trimers containing various oligopeptides and found that at least a hexapeptide to a nanopeptide, that is, an oligopeptide with a hydrophobicity index log P (P = [solute]_{n-octanol} / [solute]_{water}) of at least 2 should be employed as a hydrophobic group in the trimer to obtain stable micelles with a low critical micelle concentration (<10 mg/L).

Also in consideration of the fact that a drug carrier for intravenous injection must have a high LCST, the present inventors have elaborated optimization of the cyclic phosphazene trimer having a high LCST, preferably an LCST higher than 50°C by variation of the hydrophilicity of polyethylene glycol and found that when a hexapeptide to a nanopeptide is introduced as a hydrophobic group into the cyclic phosphazene trimer, the LCST of the phosphazene micelles is maintained at 50°C or higher only when the cyclic phosphazene trimer has as a hydrophilic group of at least a polyethylene glycol (log P < -2.8) in which the average degree of polymerization of polyethylene oxide ranges from 17 (average molecular weight 780) to 45 (average molecular weight 2000).

The oligopeptide suitable for use in the amphiphilic cyclic phosphazene trimer of Chemical Formula 1 according to the present invention is a hexapeptide or nanopeptide containing at least one hydrophobic amino acid selected from the group consisting of glycine (Gly), phenylalanine (Phe), leucine (Leu), isoleucine (Ile), alanine (Ala) and valine (Val).

Preferred examples of the alkylester or arylester include methylester, ethylester and benzylester, but are not limited thereto.

Examples of the oligopeptide ester include glycylphenylalanylleucylglycylphenylalanylleucylethylester (GlyPheLeuGlyPheLeuEt), glycylphenylalanylleucylglycylphenylalanylleucylbenzylester (GlyPheLeuGlyPheLeuBz), and glycylphenylalanylleucylglycylphenylalanylleucylglycylphenylalanylleucylethylester (GlyPheLeuGlyPheLeuGlyPheLeuEt), but are not limited thereto.

Illustrative, non-limiting examples of the amphiphilic cyclic phosphazene trimers containing the oligopeptide in accordance with Chemical Formula 1 include tris(methoxy polyethyleneglyco1780)tris(glycylphenylalanylleucylglycylphenylalanylleucylethylester)cyclotri phosphazene ([NP(MPEG780)(GlyPheLeuGlyPheLeuEt)]₃), tris(methoxypolyethylene glycol780)tris(glycylphenylalanylleucylglycylphenylalanylleucylbenzylester)cyclotriphosphaze ne ([NP(MPEG780)(GlyPheLeuGlyPheLeuBz)]₃), tris(methoxypolyethyleneglycol 1000)tris(glycylphenylalanylleucylglycylphenylalanylleucylbenzylester)cyclotriphosphazene ([NP(MPEG1000)(GlyPheLeuGlyPheLeuBz)]₃), and tris(methoxypolyethyleneglycol 2000)tris(glycylphenylalanylleucylglycylphenylalanylleucylglycylphenylalanylleucylethylester) cyclotriphosphazene([NP(MPEG2000)(GlyPheLeuGlyPheLeuGlyPheLeuEt)]₃).

The amphiphilic cyclic phosphazene trimer represented by Chemical Formula 1 can be synthesized from hexachlorocyclotriphosphazene [(N=PCl₂)₃] represented by Chemical Formula 2. For example, 1 mole of hexachlorocyclotriphosphazene of Chemical Formula 2 is subjected to stepwise substitution reactions with 3 moles of sodium or potassium salt of methoxy polyethylene glycol having an average degree of polymerization (n) of polyethylene glycol of from 17 (average molecular weight 780) to 45 (average molecular weight 2000), as a hydrophilic group, and then with 3 moles of C₁₋₆ alkylester or benzylester of hexapeptide to nanopeptide as a hydrophobic group.

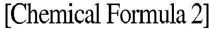

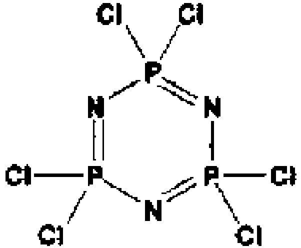

In detail, the synthesis may be achieved as follows.

First, monomethoxy polyethylene glycol with an average degree of polymerization (n) of polyethylene glycol of from 17 to 45 is reacted with an excess sodium or potassium metal piece to give a sodium or potassium salt of methoxy polyethylene glycol represented by the following Chemical Formula 3. Any solvent may be used for this reaction. For example, tetrahydrofuran, benzene or toluene may be used. This reaction may be performed by refluxing for about 5 hrs in an inert atmosphere (e.g., argon gas).

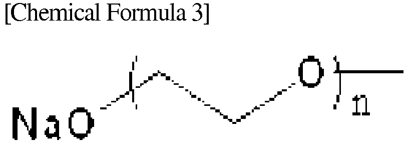

At below 0 °C, 3 equivalents of the obtained methoxy polyethylene glycol sodium or potassium salt were reacted with 1 mole (6 equivalents) of the cyclic hexachlorophosphazene trimer of Chemical Formula 1 to give a phosphazene trimer intermediate containing 3 equivalents of each of methoxy polyethylene glycol and chlorine. So long as it is not inhibitory of the reaction, any solvent may be employed. Preferably, a solvent selected from among tetrahydrofuran, toluene, chloroform, and a combination thereof may be employed.

Thereafter, the cyclic phosphazene intermediate is reacted with 3 moles of a C₁₋₆ alkylester or benzylester of a hexapeptide to a nanopeptide to afford the desired product, that is, the amphiphilic phosphazene trimer of Chemical Formula 1. In this regard, no particular limitations are imparted to the molar ratio between the reactants. Typically, 3∼5 equivalents of the C 1-6 alkylester or benzylester of a hexapeptide to a nanopeptide may be employed for 1 mole of the cyclic phosphazene intermediate. In addition, the reaction may be carried out in the presence of a base promotive of a nucleophilic substitution reaction, for example, in the presence of triethylamine. Any solvent may be used, if it is not inhibitory of the nucleophilic substitution reaction. Preferably, the solvent useful in this reaction may be selected from among tetrahydrofuran, benzene, toluene, chloroform, and a combination thereof. This reaction may be carried out at room temperature to 70°C for about 24∼72 hrs, and then at 40∼60°C for about 1∼ 4 days under reflux.

After completion of the reaction, the product of Chemical Formula 1 is separated and purified from the reaction mixture. First, precipitates produced as by-products are removed from the reaction mixture by centrifugation or filtration. The supernatant or filtrate is concentrated in a vacuum rotary evaporator and then washed three times with water. The organic layer is dried over a desiccant (e.g., MGSO4), followed by filtration in a vacuum. The filtrate is again concentrated in a vacuum, and separated and purified by chromatography to afford the pure phosphazene trimer of Chemical Formula 1 as a solid.

In accordance with another aspect thereof, the present invention addresses a pharmaceutical formulation of hydrophobic drugs by micelle-encapsulation using the amphiphilic cyclic phosphazene trimer.

As used in the context of the pharmaceutical formulation of hydrophobic drug of the present invention, the term "hydrophobic drug" refers to a drug which is sparingly dissolved in water. Any water-insoluble drug may be used, and preferred is a water-insoluble drug that needs to be formulated into an intravenous dosage form. Examples of the hydrophobic drugs useful in the present invention include, but are not limited to, anti-cancer agents such as docetaxel, paclitaxel, etc., anesthetic agents such as propofol, and peptide and protein drugs.

The pharmaceutical formulation of hydrophobic drugs by micelle-encapsulation using the amphiphilic cyclic phosphazene trimer in accordance with the present invention allows physicochemically stable solid products such as a powder, which can greatly improve the conveniences of drug storage and applications.

The pharmaceutical formulation of hydrophobic drugs by micelle-encapsulation using the amphiphilic cyclic phosphazene trimer may be prepared by dissolving 100 weight parts of the amphiphilic phosphazene trimer and 2∼30 weight parts of a hydrophobic drug in a common solvent such as ethanol, removing the common solvent by evaporation, and drying the residue to afford an amphiphilic cyclic phosphazene trimer micelle with the hydrophobic drug entrapped therein.

Conventionally, dialysis is one of the most widely used methods for dissolving water-insoluble drugs in water by micelle-encapsulation using amphiphilic polymers. That is, a water-insoluble drug and an amphiphilic polymer drug carrier are completely dissolved in a common organic solvent, and the solution is contained within a dialysis bag and dialyzed against water to give an aqueous micelle-type drug. However, this method is disadvantageous in that not only is it difficult to control the dialysis time and drug concentration but also an intricate process of recovering the micelle-encapsulated drug from the solution is required. In contrast, the method of the present invention is advantageous because of its simplicity.

No particular limitations are imparted to the common solvent used in the method for preparing a pharmaceutical formulation by micelle-encapsulation using the amphiphilic cyclic phosphazene trimer in accordance with the present invention. For example, an alcohol such as ethanol, methanol and the like, acetone, chloroform, ethyl acetate, chlorobenzene, acetonitrile or a combination thereof may be used.

### Mode for Invention

A better understanding of the present invention may be obtained through the following examples which are set forth to illustrate, but are not to be construed as limiting the present invention.

In the following examples, elemental analysis of carbon, hydrogen and nitrogen was performed using a Perkin-Elmer CHN analyzer. Proton nuclear magnetic resonance (NMR) spectra were obtained using a Bruker DPX-250 NMR Spectrometer while phosphorus NMR spectroscopy was carried out using Varian Gemini-500 NMR Spectrometer. Size distributions of nanoparticles in aqueous solutions were measured using Malvern Zetasizer (Nano-ZS).

EXAMPLE 1: Synthesis of tris(methoxy polyethylene glycol 780) tris(glycylphenylalanylleucylglycylphenylalanylleucylethylester)cyclotriphosphazene, [NP(MPEG780)(GlyPheLeuGlyPheLeuEt)]₃.

Methoxy polyethylene glycol with an average molecular weight of 780 (8.11 g, 10.4 mmol) was dehydrated using a Dean Stark apparatus in a toluene solvent, and reacted with a sodium piece (0.26 g, 11.4 mmol) for 12 hrs in an argon atmosphere under reflux to give a methoxy polyethylene glycol sodium salt solution. This salt solution was slowly dropwise added to a solution of hexachlorocyclotriphosphazene (1.00 g, 2.88 mmol) in anhydrous tetrahydrofuran in an ice bath (0°C). After removal from the ice bath, a solution of triethylamine (2.61 g, 25.8 mmol) and hexapeptide ethylester HClGlyPheLeuGlyPheLeuEt (8.05 g, 11.2 mmol) in chloroform (100 ml) was added to and reacted with the cyclotriphosphazene intermediate at 70°C for 48 hrs. Byproduct precipitates (Et₃NHCl or NaCl) were removed by centrifugation or filtration, and the filtrate was concentrated in a vacuum. The concentrate was again dissolved in methanol and concentrated in a vacuum. This procedure was repeated two or three times. The resulting concentrate was dissolved in 100 ml of methanol and dialyzed within a dialysis bag (cutoff Mw: 1000) for 24 hrs, and against water for an additional 24 hrs. To remove other minor isomers, column chromatography (alumina, 50-200 mesh, neutral, MC/MeOH=97:3) was performed, followed by vacuum distillation to afford a desired phosphazene product [NP(MPEG780) (GlyPheLeuGlyPheLeuEt)]3 (yield, 64.0%).

Empirical formula: C₂₁₃H₃₆₆N₂₁O₇₅P₃.

Molecular weight = 4483.9

Elemental analysis

Calculated: C, 57.06; H, 7.55; N, 6.56. Found: C, 56.6; H, 8.21; N, 6.33.

¹H NMR (CDCl₃, δ in ppm): 0.69-0.87 (m, 36H, 2(Leu-(CH₃)₂)), 1.14 (t, 9H, Leu-OCH₂CH₃), 1.31-1.62 (m, 18H, 2(Leu-CHCH₂)), 2.60-3.09 (b, 12H, 2(Phe-CH₂)), 3.68-3.96 (b, 12H, 2(Gly-CH₂)), 4.0-4.1 (m, 6H, Leu-OCH₂CH₃), 4.17-4.44 (m, 6H, 2(Leu-CH)), 4.5-4.8 (b, 6H, 2(Phe-CH)), 7.14-7.23 (m, 30H, Phe-C₅H₅), 7.9-8.54 (m, 18H, NHCO)

³¹P NMR(CDCl₃, δ in ppm): 22.4.

Lower critical solution temperature (LCST): 72.0 °C.

EXAMPLE 2: Synthesis of tris(methoxy polyethylene glycol780) tris(glycylphenylalanylleucylglycylphenylalanylleucylbenzylester)cyclotriphosphazene, [NP(MPEG780)(GlyPheLeuGlyPheLeuBz)]₃.

The following amphiphilic cyclic phosphazene trimer [NP(MPEG780)(GlyPheLeuGlyPheLeuBz)]₃ was synthesized in the same manner as in Example 1, with the exception that hexapeptide benzylester HclGlyPheLeuGlyPheLeuBz was used instead of hexapeptide ethylester HclGlyPheLeuGlyPheLeuEt (Yield: 45.7%).

Empirical formula: C₂₄₃H₄₀₂N₂₁O₈₁P₃.

Molecular weight: 5006.8

Elemental analysis: Calculated: C, 58.29; H, 8.09; N,5.87. Found: C, 58.73; H, 8.09; N, 6.23.

¹H NMR (250MHz, DMSO, 25°C): δ= 0.73-0.87 (m, 36H, 2(Leu-(CH₃)₂)), 1.2-1.56 (m, 18H, 2(Leu-CH₂CH)), 2.71-2.3 (m, 12H, 2(Phe-CH₂)), 3.24 (s, 9H MPEG-OCH₃), 3.79 (d, 12H, 2(Gly-CH₂)), 4.3 (b, 6H, 2(Leu-CH)), 4.61 (b, 6H, 2(Phe-CH)), 5.11(d, 6H, C₆H₅-CH₂), 7.22 (s, 30H, 2(Phe-C₆H₅)), 7.5 (s, 15H, O-CH₂-C₆H₅), 7.9-8.54 (m, 18H; NHCO)

³¹P NMR(CDCl₃, ppm): δ= 22.7

LCST = 66 °C

EXAMPLE 3: Synthesis of tris(methoxy polyethylene glycol 1000) tris(glycylphenylalanylleucylglycylphenylalanylleucylbenzylester)cyclotriphosphazene, [NP(MPEG1000)(GlyPheLeuGlyPheLeuBz)]₃.

The following amphiphilic cyclic phosphazene trimer [NP(MPEG1000)(GlyPheLeuGlyPheLeuBz)]₃ was synthesized in the same manner as in Example 1, with the exception that methoxypolyethyleneglycol with a molecular weight of 1000 and hexapeptide benzylester HClGlyPheLeuGlyPheLeuBz were used instead of methoxy polyethylene glycol with a molecular weight of 780 and hexapeptide ethylester HClGlyPheLeuGlyPheLeuEt, respectively (yield 50. 7 %).

Empirical formula: C₂₅₈H₄₂₃N₂₁O₉₀P₃.

Molecular weight: 5322.2

Elemental analysis: Calculated: C, 57.80; H, 8.12; N, 5.49. Found: C, 57.63; H, 8.10; N, 5.43.

¹H NMR (250MHz, DMSO, 25°C): δ= 0.73-0.87 (m, 36H, 2(Leu-(CH₃)₂)), 1.2-1.56 (m, 18H, 2(Leu-CH₂CH)), 2.71-2.3 (m, 12H, 2(Phe-CH₂)), 3.24 (s, 9H, MPEG-OCH₃), 3.37-3.5 (m, 264H, MPEG1000-OCH₂CH₂), 3.79 (d, 12H, 2(Gly-CH₂)), 4.3 (b, 6H; 2(Leu-CH)), 4.61 (b, 6H; 2(Phe-CH)), 5.11(d, 6H; C₆H₅-CH₂), 7.22 (s, 30H, 2(Phe-C₆H₅)), 7.5 (s, 15H, O-CH₂-C₆₆H₅), 7.9-8.54 (m, 18H; NHCO)

³¹P NMR(CDCl_{C}, ppm): δ= 22.9

LCST = 70 °C.

EXAMPLE 4: Synthesis of tris(methoxy polyethylene glycol 2000) tris(glycylphenylalanylleucylglycylphenylalanylleucylglycylphenylalanylleucylethylester) cyclotriphosphazene, [NP(MPEG2000)(GlyPheLeuGlyPheLeuGlyPheLeuEt)]₃.

The amphiphilic cyclic phosphazene trimer [NP(MPEG2000) (GlyPheLeuGlyPheLeuGlyPheLeuEt)]₃ was synthesized in the same manner as in Example 1, with the exception that methoxy polyethylene glycol with a molecular weight of 2000 and nanopeptide ethylester HClGlyPheLeuGlyPheLeuGlyPheLeuEt were used instead of methoxy polyethylene glycol with a molecular weight of 780 and hexapeptide ethylester HClGlyPheLeuGlyPheLeuEt, respectively (Yield: 39.7%).

Empirical formula: C₄₃₂H₇₇₁N₃₀O₁₆₈P₃.

Molecular weight: 9166.77

Elemental analysis: calculated: C, 56.60; H, 8.48; N, 4.58. Found: C, 56.33; H, 8.79; N, 4.37.

¹H NMR (250MHz, DMSO, 25°C): δ= 0.73-0.87 (m, 54H 3(Leu-(CH₃)₂)), 1.10 (t, 9H, Leu-OCH₂CH₃) 1.2-1.56 (m, 27H, 3(Leu-CH₂CH)), 2.71-2.3 (m, 18H, 3(Phe-CH₂)), 3.24 (s, 9H, MPEG2,000-OCH₃), 3.37-3.5 (m, 540H, MPEG2,000-OCH₂CH₂), 3.79 10 (d, 18H, 3(Gly-CH₂)), 4.0-4.1 (m, 6H, Leu-OCH₂CH₃), 4.3 (b, 9H, 3(Leu-CH)), 4.61 (b, 9H, 3(Phe-CH)), 7.22 (s, 45H, 3(Phe-C₆H₅)), 7.9-8.54 (m, 18H; NHCO)

³¹P NMR(CDCl₃, ppm): δ= 22.6

LCST = 96 °C.

EXAMPLE 5: Preparation of docetaxel anticancer agent micelle-encapsulated by [NP(MPEG780)(GlyPheLeuGlyPheLeuEt)]₃.

25 mg of docetaxel and 100 mg of the phosphazene trimer synthesized in Example 1 were completely dissolved in 10 ml of absolute ethanol, and then ethanol was slowly evaporated to dryness using a vacuum rotary evaporator, followed by completely removing the residual solvent by vacuum drying for 30 min to 1 hr to afford phosphazene trimer micelles in a solid phase with the docetaxel anticancer agent entrapped therein.

EXAMPLES 6 AND 7: Preparation of docetaxel anticancer agent micelle-encapsulated by [NP(MPEG780)(GlyPheLeuGlyPheLeuEt)]₃.

Phosphazene trimer micelles with docetaxel entrapped therein were prepared in the same manner as in Example 5, with the exception that the ingredients were used as shown in Table 1, below.

TABLE 1

**[Table 1]**

| | Phosphazene Trimer of Example 1 (mg) | Docetaxel (mg) | Absolute EtOH (ml) |
|---|---|---|---|
| Ex. 5 | 100 | 25 | 10 |
| Ex. 6 | 100 | 12 | 10 |
| Ex. 7 | 100 | 5 | 5 |

EXAMPLES 8 TO 10: Preparation of paclitaxel anticancer agent micelle-encapsulated by [NP(MPEG780)(GlyPheLeuGlyPheLeuEt)]₃.

Phosphazene trimer micelles with paclitaxel entrapped therein were prepared in the same manner as in Example 5, with the exception that the ingredients were used as shown in Table 2, below.

TABLE 2

**[Table 2]**

| | phosphazene trimer(mg) of Ex. 1 | Paclitaxel(mg) | Absolute EtOH (ml) |
|---|---|---|---|
| Ex. 8 | 100 | 25 | 10 |
| Ex. 9 | 100 | 12 | 10 |
| Ex. 10 | 100 | 5 | 5 |

EXAMPLE 11: Preparation of propofol solution by micelle-encapsulation using [NP(MPEG780)(GlyPheLeuGlyPheLeuEt)]₃.

5 mg of propofol and 45 mg of the phosphazene trimer synthesized in Example 1 was dissolved in 10 ml of absolute ethanol, and then ethanol was slowly evaporated in a vacuum rotary evaporator, followed by completely removing the residual solvent by vacuum drying for 10 to 20 min. The residue was dissolved in 1 ml of distilled water to afford a transparent 0.5 % propofol injection solution.

EXAMPLES 12 TO 14: Preparation of docetaxel anticancer agent micelle-encapsulated by [NP(MPEG780)(GlyPheLeuGlyPheLeuBz)]₃.

Docetaxel micelle-encapsulated by the phosphazene trimer prepared in Example 2 in the same manner as in Example 5 with the exception that the ingredients were used as shown in Table 3, below.

TABLE 3

**[Table 3]**

| | Phosphazene trimer (mg) of Ex. 2 | Docetaxel (mg) | Absolute EtOH (ml) |
|---|---|---|---|
| Ex. 12 | 100 | 25 | 10 |
| Ex. 13 | 100 | 12 | 10 |
| Ex. 14 | 100 | 5 | 5 |

EXAMPLES 15 TO 17: Preparation of docetaxel anticancer agent micelle-encapsulated by [NP(MPEG1000)(GlyPheLeuGlyPheLeuBz)]₃

Docetaxel micelle-encapsulated by the phosphazene trimer prepared in Example 3 in the same manner as in Example 5 with the exception that the ingredients were used as shown in Table 4, below.

TABLE 4

**[Table 4]**

| | Phosphazene trimer (mg) of Ex. 3 | docetaxel(mg) | Absolute EtOH (ml) |
|---|---|---|---|
| Ex. 15 | 100 | 25 | 10 |
| Ex. 16 | 100 | 12 | 10 |
| Ex. 17 | 100 | 5 | 5 |

EXAMPLES 18 TO 20: Preparation of docetaxel anticancer agent micelle-encapsulated by [NP(MPEG2000)(GlyPheLeuGlyPheLeuGlyPheLeuEt)]₃

Docetaxel micelle-encapsulated by the phosphazene trimer prepared in Example 4 in the same manner as in Example 5 with the exception that the ingredients were used as shown in Table 5, below.

TABLE 5

**[Table 5]**

| | Phosphazene trimer (mg) of Ex. 4 | Docetaxel(mg) | Absolute EtOH (ml) |
|---|---|---|---|
| Ex. 18 | 100 | 25 | 10 |
| Ex. 19 | 100 | 12 | 10 |
| Ex. 20 | 100 | 5 | 5 |

EXAMPLE 21: Comparison between docetaxel formulations of the present inventive [NP(MPEG780)(GlyPheLeuGlyPheLeuEt)]₃ and known [NP(MPEG350)(GlyPheLeuGlyEt)]₃

Docetaxel was formulated with the phosphazene trimer of Example 1 of the present invention and the phosphazene trimer of Example 1 of Korean Patent No. 0567397(Sohn, Youn Soo et al.) in the same manner as in Example 5 of the present invention, and the two formulations were compared in micelle stability in aqueous solution and injectability in mice. First of all, the docetaxel anticancer agent formulated with the phosphazene trimer of Example 1 of the present invention was obtained in the form of powder while the docetaxel anticancer agent formulated with the phosphazene trimer of Example 1 of Korean Patent No. 0567397 was obtained as a liquid oil. Each of these two formulations was dissolved in water to form transparent aqueous solution containing 0.2 % docetaxel, and then 10 mL of each fresh solution was placed in a 20 mL transparent vial, which was observed at room temperature under a typical interior light. Most of docetaxel was found to precipitate in the vial containing the formulated solution of Example 1 of Korean Patent No. 0567397 after 3 days, probably due to instability of the micelles formed from the trimer bearing the tetrapeptide, whereas no precipitate was found even after 3 months in the vial containing the formulation prepared with the trimer bearing hexapeptide of Example 1 of the present invention. In addition, each of the aqueous solutions was attempted to inject intravenously at a dose of 10 mg/kg to 3 ICR mice. The solution of Example 1 of the present invention (LCST= 72°C) was readily administered through the tail vein, whereas the solution of Example 1 of Korean Patent No. 0567397 (LCST= 36°C) could not be completely infused as the solution increased in viscosity due to its low LCST from the start of the infusion.

EXPERIMENTAL EXAMPLE 1: Comparison of blood metabolism between phosphazene micelle-encapsulated docetaxel and Taxotere®

To groups of five male Sprague-Dawley (SD) rats (230∼250 g), the phosphazene micelle-encapsulated docetaxel of Example 6 was i.v. injected at a docetaxel dose of 5 mg/kg over 3 min. For comparison, Taxotere® was administered in the same way. Blood samples (100 µl) were taken at predetermined times (0, 0.08, 0.25, 0.5,1, 2, 3, 4, 6, 8,12, 24, 36, 48 hrs) after administration, and plasma was separated from each sample by centrifugation and stored at -20°C before HPLC analysis. From the plasma docetaxel concentration vs. time profile, pharmacokinetic parameters were calculated using the WinNonlin program, and are summarized in Table 6, below.

TABLE 6

**[Table 6]**

| Metabolism Parameters | Taxotere | Example 6 |
|---|---|---|
| | Mean±S.D | Mean±S.D. |
| C₀ (ug/ml) | 5.32±1.58 | 4.07±0.92 |
| AUClast(ug*hr/ml) | 0.360±0.116 | 0.664±0.050 |
| t_{1/2}(hr) | 0.396±1.11 | 0.711±0.796 |
| Ke(1/hr) | 8.05±3.65 | 1.78±0.992 |
| Vz(L) | 1.24±2.21 | 1.67±1.55 |
| Cl(L/hr) | 3.38±1.00 | 1.86±0.281 |

*C₀: Initial concentration, AUC: Area under plasma-time curve, t_{1/2}: half life, Ke: elimination constant, Vz: distribution volume, Cl: clearance rate

As is seen from the data of Table 6, the *in vivo* half-life (t1/2 = 0.711 hrs) of the polymer micelle type docetaxel anticancer agent of Example 6 is almost twice compared with that of the currently clinically used Taxotere® (t1/2 = 0.396 hrs). Also, a two-fold greater AUC (area under the curve), which accounts for bioavailability, was found in the polymer micelle type docetaxel anticancer agent of Example 6 (0.664) than in Taxotere® (0.360). In consideration of the fact that taxane anticancer agents are highly hydrophobic and are rapidly cleared by serum transport proteins, unlike other anticancer agents such as a platinum complex, a two-fold increase in serum half life and bioavailability is a significant advance.

EXPERIMENTAL EXAMPLE 2: Comparison of blood metabolism between phosphazene micelle-encapsulated paclitaxel and Taxol

Experiments for blood metabolism were conducted in the same manner as in Experiment Example 1, with the exception that micelle-encapsulated paclitaxel of Example 8 and Taxol formulated with Cremophor EL (Bristolmyer) were used instead. The results are summarized in Table 7 below. As can be seen in Table 7, the micelle-encapsulated paclitaxel of the present invention was two-fold increased in both serum half-life and AUC, compared to Taxol.

TABLE 7

**[Table 7]**

| Metabolism Parameter | Taxol | Example 8 |
|---|---|---|
| | Mean±S.D. | Mean±S.D. |
| C₀ (ug/ml) | 2.70±0.65 | 1.53±0.15 |
| AUClast (ug*hr/ml) | 1.97±0.31 | 2.97±0.59 |
| t_{1/2} (hr) | 3.54±2.46 | 6.29±2.51 |
| Ke(1/hr) | 0.275±0.165 | 0.133±0.077 |
| Vz(L) | 2.78±1.74 | 4.47±2.01 |
| Cl(L/hr) | 0.56±0.063 | 0.51±0.015 |

*C₀: Initial concentration, AUC: Area under plasma-time curve, t_{1/2}: half life, Ke: elimination constant, Vz: distribution volume, Cl: clearance rate

EXPERIMENTAL EXAMPLE 3: Comparison of *in vivo* anticancer activity of phosphazene micelle-encapsulated docetaxel and Taxotere® in nude mouse model

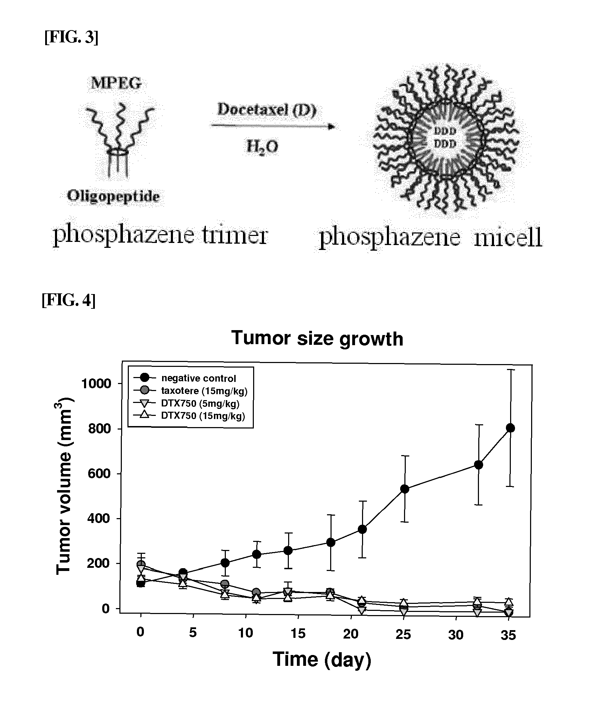

Nude mice CAnN.Cg-Foxn1nu/CrljOri were divided into groups each consisting of 5 mice. After implantation into the nude mice, the breast cancer cell line MDA-MB-231 was grown until a tumor increased to a size of 80-100 mm³. The phosphazene micelle-encapsulated docetaxel of Example 6 was administered at a dose of 5 mg/kg and 15 mg/kg to the mice three times respectively on day 1, 5 and 9 after implantation, followed by monitoring cancer cell growth for 5 weeks. The results are given in FIG. 4. For comparison, an excipient was used as a placebo while Taxotere® was used at a dose of 15 mg/kg, known as an optimal dose, serving as a positive control.

In the plot of FIG. 4, Taxotere®, as expected, exhibited complete regression of cancer tissues at the optimal dose of 15 mg/kg, while the phosphazene micelle-encapsulated docetaxel (DTX) of the present invention exerted perfect therapeutic effects on the cancer tissues at a dose of 5 mg/kg as well as 15 mg/kg. Such animal experimental data indicate that phosphazene micelle-encapsulated docetaxel may reduce the therapeutic dose of docetaxel to a 1/3 level, compared to Taxotere®, showing an exceptional effect. This result is coincident with the previous data of the blood metabolism in which the bioavailability parameter (AUC) increased two fold. If the clinical dose of docetaxel can be reduced by two thirds, many ripple effects are expected because a great reduction can be brought about not only in the side effects by docetaxel but also in the cost of therapy. In addition, the experimental result of anti-cancer activity was coincident with the results of acute cytotoxicity in the following Experimental Example 4.

EXPERIMENTAL EXAMPLE 4: Comparison of cytotoxicity between micelle-encapsulated docetaxel and Taxotere®

In order to compare the spectrum and efficacy of anticancer therapy between the polymer micelle-encapsulated docetaxel of the present invention and the currently used anticancer drug Taxotere®, experiments for *in vitro* anticancer activity were conducted in 8 different human cancer cell lines according to the method known in the art (Rita Song et al., J. Control. Release 105 (2005) 142-150), and the results are summarized in Table 8, below. As shown in Table 8, the phosphazene micelle-encapsulated docetaxel was generally lower in *in vitro* cytotoxicity (IC₅₀), compared to Taxotere®, irrespective of the molecular weight of PEG. This is believed to be attributed to the fact that the docetaxel drug molecules are trapped within the strong phosphazene micelle core so that they are brought into contact with cancer cells in a delayed pattern. However, the phosphazene micelle-encapsulated docetaxel and Taxotere® were very similar in the spectrum of anticancer therapy.

TABLE 8

**[Table 8]**

| Tumor cells | *In vitro* cytotoxicity (IC₅₀, nM) | | |
|---|---|---|---|
| | Taxotere® | Example 6 | Example 16 |
| HCT-15(colon) | 34.1 ±0.08 | 54.7 ±17.2 | 58.4 ±13.9 |
| A549(lung) | 5.85 ±1.85 | 8.81 ±1.41 | 10.4 ±1.46 |
| PC3(prostate) | 9.80 ±2.09 | 12.6 ±1.22 | 13.7 ±1.15 |
| SNU638(stomach) | 0.748 ±0.09 | 3.65 ±0.83 | 5.27 ±0.08 |
| SK-OV-3(ovary) | 9.21 ±0.59 | 14.4 ±1.42 | 14.6 ±2.73 |
| A431(epidermoid) | 4.90 ±2.33 | 10.8 ±2.73 | 14.5 ±5.17 |
| MCF-7(breast) | 4.24 ±0.52 | 8.74 ±0.40 | 9.85 ±0.225 |
| MES-SA(cervical) | 31.1 ±10.2 | 26.8 ±8.97 | 36.8 ±9.41 |

EXPERIMENTAL EXAMPLE 5: Comparison of acute toxicity between phosphazene micelle-encapsulated docetaxel and Taxotere®

Male ICR mice with an age of 6 weeks, each weighing 20-22 g, were adapted at 20 °C and 60 % humidity for 5 days before experiments were conducted according to the OECD guide line 423. Test materials were dissolved in physiological saline. Taxotere® was intravenously injected at doses of 5, 30, 50, and 100 mg/kg into each group of three mice while the phosphazene micelle-encapsulated docetaxel of Example 6 containing 10 % docetaxel were administered at doses of 5, 50, 300, 500, 1000, and 2000 mg/kg. The mice were monitored for body weight change and survival for 2 weeks, after which biopsies were performed and the value of LD₅₀ was calculated according to the OECD guide line 425.

When administered with Taxotere®, all of the three mice survived at a dose of 5 mg/kg while two of the three were dead at 30 mg/kg, and all were dead at 50 and 100 mg/kg. Accordingly, the LD₅₀ value of Taxotere® was calculated to be about 28 mg/kg. As for the phosphazene micelle-encapsulated docetaxel containing 10 % docetaxel, their LD₅₀ was calculated to be about 750 mg/kg on the basis of the data that all the mice survived at up to 500 mg/kg whereas all the mice administered at 1000 or 2000 mg/kg were dead. Therefore, the LD₅₀ value of the micelle-encapsulated docetaxel is calculated to be 75 mg/kg based on docetaxel. Thus the LD₅₀ value of phosphazene micelle-encapsulated docetaxel of Example 6 was increased approximately 3 times (75 mg/kg) compared to that of Taxotere® which was formulated with polysorbate 80 (28 mg/kg). Such a remarkable improvement of acute toxicity is believed to be attributed to the fact that Taxotere®, a surfactant micelle, is disrupted immediately after injection so that docetaxel is rapidly released into the blood stream while the phosphazene micelle-encapsulated docetaxel is stable in the blood stream so that docetaxel is released in a sustained manner, thereby increasing bioavailability and pharmaceutical efficacy with significantly improved toxicity.

EXPERIMENTAL EXAMPLE 6: Comparison of phosphazene micelle-encapsulated propofol and Diprivan®

Sprague-Dawley (SD) rats (female, 16 weeks old, 300∼340g) were divided into groups, each consisting of three rats, and IV cannulation was performed on each rat to provide a venous access through the jugular vein. Using a microprocessor-controlled syringe pump, test materials were infused at a rate of 10 mg/kg/min through the venous route. Anesthetic induction was evaluated in the order of the following items, and the amount of propofol infused to the end point was determined to be the dose.

- Loss of observatory activity (Stunned)

- Loss of righting reflex

- Loss of lash reflex

- Loss of leg withdrawal in response to stimuli to the toe every 10 sec.

Experimental results are summarized in Table 9, below. As is seen from the data of Table 9, it took a slightly longer time to anesthetize rats with the phosphazene micelle-encapsulated propofol of Example 11 than the commercially available propofol product Diprivan®, a lipid emulsion for injection, indicating that the conventional propofol in a lipid emulsion form is freed from the emulsion immediately after injection and reacts with pain receptors in the blood whereas the propofol entrapped in the phosphazene micelles are not released into blood immediately after injection but are absorbed in the micelle form into tissues, thus delaying the anesthesia. Therefore, the propofol entrapped in the phosphazene micelles is shorter in reaction time with pain receptors than is the propofol in a lipid emulsion, thus making a great contribution to pain reduction.

TABLE 9

**[Table 9]**

| Micelle-type drug (PPF, n = 3) | | | | | | | Diprivan® (n = 30) | |
|---|---|---|---|---|---|---|---|---|
| Demography | | | | | | | | |
| ID | Unit | 1 | 2 | 3 | Mean | SD | Mean | SD |
| Test date | | 2011-03-16 | 2011-03-16 | 2011-03-16 | | | | |
| Age | weeks | 16 | 16 | 16 | 16.00 | 0.00 | | |
| Sex | | Female | Female | Female | | | | |
| BW | kg | 0.34 | 0.305 | 0.311 | 0.32 | 0.02 | | |
| Rate | mg/min | 3.4 | 3.05 | 3.11 | 3.19 | 0.19 | | |

| Anesthetic induction | | | | | | | | |
|---|---|---|---|---|---|---|---|---|
| Dose | mg/kg | 22.5 | 38.3 | 40.8 | 33.8 | 9.94 | 20.5 | 4.4 |
| Stunned | sec | 50 | 65 | 25 | 46.6 | 20.2 | 40 | 9 |
| Loss of righting reflex | sec | 60 | 100 | 100 | 86.6 | 23.0 | 55 | 12 |
| Loss of | sec | 70 | 140 | 180 | 130 | 55.6 | 89 | 17 |
| lash reflex | | | | | | | | |
| Loss of leg withdrawal | sec | 135 | 230 | 245 | 203 | 59.6 | 123 | 26 |

## Claims

1. An amphiphilic cyclic phosphazene trimer, represented by the following Chemical Formula 1:
[Chemical Formula 1] [N=P(MPEG)(oligopeptide ester)]₃
wherein MPEG is a methoxy polyethylene glycol with an average degree of polymerization (n) of ethylene oxide (CH₂CH₂O)ₙ ranging from 17 (average molecular weight of 780) to 45 (average molecular weight of 2000), and the oligopeptide ester is a C₁₋₆ alkylester or C₇₋₁₃ arylalkylester of a hexapeptide or a nanopeptide.

2. The amphiphilic cyclic phosphazene trimer of claim 1, wherein the oligopeptide contains a hydrophobic amino acid selected from the group consisting of glycine (Gly), phenylalanine (Phe), leucine (Leu), isoleucine (Ile), alanine (Ala), valine (Val), and a combination thereof.

3. The amphiphilic cyclic phosphazene trimer of claim 1, wherein the oligopeptide ester is glycylphenylalanylleucylglycylphenylalanylleucylethylester (GlyPheLeuGlyPheLeuEt), glycylphenylalanylleucylglycylphenylalanylleucylbenzylester (GlyPheLeuGlyPheLeuBz) or glycylphenylalanylleucylglycylphenylalanylleucylglycylphenylalanylleucylethylester (GlyPheLeuGlyPheLeuGlyPheLeuEt).

4. The amphiphilic cyclic phosphazene trimer of claim 1, being selected from among tris(methoxypolyethyleneglycol780)tris(glycylphenylalanylleucylglycylphenylalanylleucylethyl ester)cyclotriphosphazene ([NP(MPEG780)(GlyPheLeuGlyPheLeuEt)]₃), tris(methoxypolyethyleneglycol780)tris(glycylphenylalanylleucylglycylphenylalanylleucylbenz ylester)cyclotriphosphazene ([NP(MPEG780)(GlyPheLeuGlyPheLeuBz)]₃), tris(methoxypolyethyleneglycol1000)tris(glycylphenylalanylleucylglycylphenylalanylleucylben zylester)cyclotriphosphazene ([NP(MPEG1000)(GlyPheLeuGlyPheLeuBz)]₃), and tris(methoxypolyethyleneglycol2000)tris(glycylphenylalanylleucylglycylphenylalanylleucylglyc ylphenylalanylleucylethylester)cyclotriphosphazene ([NP(MPEG2000)(GlyPheLeuGl yPheLeuGlyPheLeuEt)]₃).

5. A pharmaceutical formulation of a hydrophobic drug, comprising micelles of the amphiphilic cyclic phosphazene trimer of claim 1, and a hydrophobic drug entrapped in the micelles.

6. A pharmaceutical formulation of a hydrophobic drug of claim 5, wherein the hydrophobic drug has an anticancer activity.

7. The pharmaceutical formulation of claim 6, wherein the hydrophobic drug is docetaxel or paclitaxel.

8. A pharmaceutical formulation of a hydrophobic drug of claim 5, wherein the hydrophobic drug is an anesthetic agent.

9. The pharmaceutical formulation of a hydrophobic drug of claim 8, wherein the hydrophobic drug is propofol.

10. A method for preparing a pharmaceutical formulation in a micelle form with a hydrophobic drug entrapped therein, comprising: dissolving 100 parts by weight of the amphiphilic cyclic phosphazene trimer of claim 1, together with 2∼30 parts by weight of the hydrophobic drug, in a common solvent; evaporating the common solvent; and drying the residue to form micells of the amphiphilic cyclic phosphazene trimer with the hydrophobic drug entrapped therein.

11. The pharmaceutical formulation of a hydrophobic drug of claim 10, wherein the common solvent is selected from the group consisting of alcohol, acetone, chloroform, ethylacetate, chlorobenzene, acetonitrile, and a combination thereof.
